# FASCICULE DE BREVET EUROPEEN

(11) **EP 0 661 952 B1**
(45) Date de publication et mention de la délivrance du brevet: **06.11.1996**
(21) Numéro de dépôt: 93920886.4
(22) Date de dépôt: 15.09.1993
(51) Int. Cl.: A61C 8/00, A61B 19/00

(54) **DISPOSITIF D'EXPANSION POUR RECONSTRUCTION ORALE**
DEHNVORRICHTUNG FÜR ORALE REKONSTRUKTION
EXPANSION DEVICE FOR ORAL RECONSTRUCTION

(30) Priorité: 15.09.1992 FR 9210992
(43) Date de publication de la demande: 12.07.1995
(73) Titulaire: ROBERT, Antoine, F-92170 Vanves (FR)
(72) Inventeur: ROBERT, Antoine, F-92170 Vanves (FR)
(74) Mandataire: Schmit, Christian Norbert Marie
(86) Numéro de dépôt international: FR9300890
(87) Numéro de publication internationale: WO9406368

(56) Documents cités:
- EP-A- 0 196 821
- EP-A- 0 260 081
- FR-A- 2 608 916
- US-A- 3 343 263
- US-A- 4 671 255
- US-A- 4 719 918
- US-A- 5 074 878
- US-A- 5 137 529

## Description

La présente invention consiste en un dispositif d'expansion à base d'une pellicule de silicone qui facilite la reconstruction orale en cas de perte osseuse et le procédé d'installation dudit dispositif d'expansion sous la gencive d'un patient dans le but d'effectuer la reconstruction orale.

Lors de l'extraction d'une dent, l'os alvéolaire est graduellement réabsorbé à cause du stimulus de la perte de la pression des dents qui induit l'ossification. Au fur et à mesure de l'avancement, du processus de réabsorption, la taille de l'os se réduit, c'est à dire que l'os sur lequel sont disposés les racines dentaires commence à se rétrécir (processus alvéolaire).

L'absence d'une seule dent peut déjà déclencher des modifications dans toute l'arcade dentaire, jusqu'à provoquer un éventuel ramollissement (perte d'insertion) qui peut impliquer la perte d'autres dents. L'absence de plusieurs dents aggrave le problème. La perte osseuse qui s'ensuit finit par modifier l'apparence du patient et, selon la perte, le rendre inapte à recevoir des bridges, des implants et même un dentier.

On devrait alors procéder à plusieurs opérations chirurgicales pour reconstituer la crête alvéolaire de la mandibule ou du maxillaire.

Bien que ces procédés chirurgicaux de reconstitution aient été réalisés avec succès, ce type d'intervention présentait des inconvénients. Certains procédés comportaient l'ouverture du tissu mucopériosté sur toute la longueur de la crête alvéolaire atrophiée et l'installation ultérieure de la substance hydroxyapatite de calcium au sommet de la crête alvéolaire édentée, en même temps que le maintien de l'hydroxyapatite en place, alors que l'on suturait le délicat tissu mucopériosté, tendu, pour le remettre à sa position initiale. Cette intervention chirurgicale présentait des inconvénients, tels que la rupture du tissu mucopériosté et de déplacement de l'hydroxyapatite ainsi qu'une insuffisance dans la reconstitution de la crête alvéolaire de la mandibule ou du maxillaire.

De plus, les techniques chirurgicales conventionnelles engendraient généralement des difficultés pour le maintien des particules d'hydroxyapatite le long de la crête alvéolaire afin d'éviter qu'elles migrent vers les sillons linguaux ou les vestibules buccal et labial. Quelquefois, dans un effort pour recouvrir adéquatement les tissus, on oblitérait le vestibule buccal, rendant nécessaire une vestibuloplastie. Il y avait aussi des cas de paresthésies labiales causées par des atteintes aux nerfs mentonniers. On utilisait communément le dispositif chirurgical "Stent" pour le contrôle des particules, ce qui pouvait occasionner une érosion de la muqueuse et la déhiscence de l'hydroxyapatite à partir de la pression du "Stent".

Outre les chirurgies pour reconstruction orale connues de la technique, il existe également des dispositifs gonflables ou expansibles qui sont placés sous le périosteum dans la crête mandibulaire ou maxillaire édentée.

Ces dispositifs gonflables offrent des avantages par rapport aux chirurgies de la technique antérieure, dans la mesure où ils évitent les nécroses, les ruptures de tissus, la paresthésie du nerf mentonnier, les migrations d'hydroxyapatite, l'oblitération du vestibule buccal, l'érosion des muqueuses, la déhiscence, la formation d'hématomes, etc.

Cependant, ces dispositifs gonflables connus comportent une ou plusieurs canules par où on introduit le liquide expanseur. Alors que ce dispositif d'expansion proprement dit est placé sous le tissu subpériosté, sa canule reste en dehors de la bouche, ce qui est très inconfortable. De plus, la canule, en tant que lien entre l'intérieur de la gencive et l'extérieur, augmente le risque d'infection.

L'objectif de la présente invention est d'offrir un dispositif d'expansion similaire à ceux connus de la technique, mais qui puisse être appliqué à la bouche sans risque ni inconfort pour le patient. Dans un document US-A-4 719 918, formant le préambule de la revendication 1, il est déjà décrit un dispositif d'expansion avec une canule qui comporte, en variante, une plaque en un matériau formant valve naturelle plaquée contre une paroi d'une poche fermée formant le dispositif et destinée à recevoir une aiguille de remplissage. La valve est même placée de préférence à l'intérieur de la poche. Ce dispositif comporte en outre une protection contre la perforation de part en part pour éviter sa détérioration lors du remplissage. Cette protection est constituée par une plaque dure collée sur une face opposée de la poche par rapport à celle qui porte la valve.

Cette réalisation présente des inconvénients en ce sens que la plaque de protection est ainsi placée sur un fond de la poche et en diminue la souplesse. Une bonne souplesse est cependant nécessaire lorsque la poche est posée sur l'os au moment de l'intervention. La poche épouse alors plus facilement le relief de l'os. D'autre part l'enfoncement de l'aiguille de remplissage (tous les trois jours par exemple) ne doit se faire que dans une direction précise qui est celle présentée par l'alignement de la valve avec la plaque de protection. Ceci est quelquefois délicat compte tenu de la place de la poche dans la bouche du patient.

Pour résoudre ces problèmes, dans l'invention on réalise dans une telle poche un accrochage d'un capot protecteur directement sur la paroi qui porte la valve, à proximité de la valve, ou même de préférence sur la valve elle même, et non sur la paroi de la poche opposée à la paroi qui porte la valve. Ce capot qui a alors une forme concave et non plus une forme plate procure une plus grande tolérance d'insertion de l'aiguille de remplissage au moment de l'injection puisqu'il est plus près de cette valve. La forme concave du capot contribue au même résultat technique de protection. Dans le document cité, la tolérance d'orientation de l'aiguille au moment de l'injection est plus limitée. Ou alors la plaque de protection doit être plus grande, ce qui réduit encore la souplesse. Comme on le verra par la suite la réalisation de la poche de l'invention n'est cependant pas plus difficile par ailleurs. Elle nécessite plus d'opérations, mais ces opérations sont simples.

L'objectif ci-dessus est atteint par la présentation d'un dispositif d'expansion pour reconstruction orale constitué par une poche fermée faite d'une pellicule munie en un endroit d'une surépaisseur et d'une protection contre les perforations de part en part, caractérisé en ce que la protection comprend à l'intérieur de la poche un capot concave disposé en regard de la surépaisseur, collé directement de part et d'autre de la surépaisseur mais pas sur tout son pourtour.

Dans le document US-A-5 074 878, la protection contre les perforations se trouve en suspension dans la poche ou elle est attachée sur tout son pourtour ou sur une partie du pourtour, sans pour autant préciser que cela augmente la souplesse de la poche.

Afin de permettre une meilleure compréhension de cette invention, on trouvera ci-dessous une description détaillée ainsi que des dessins en annexe, dans lesquels:

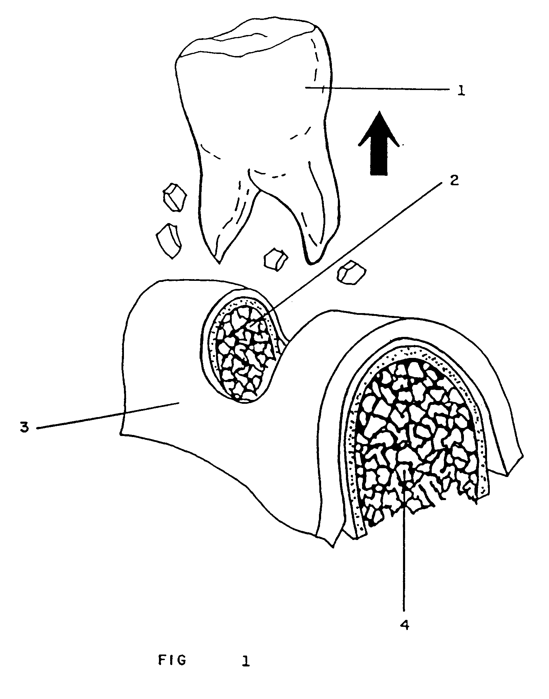

La figure 1 est une vue schématique illustrant l'extraction d'une dent;

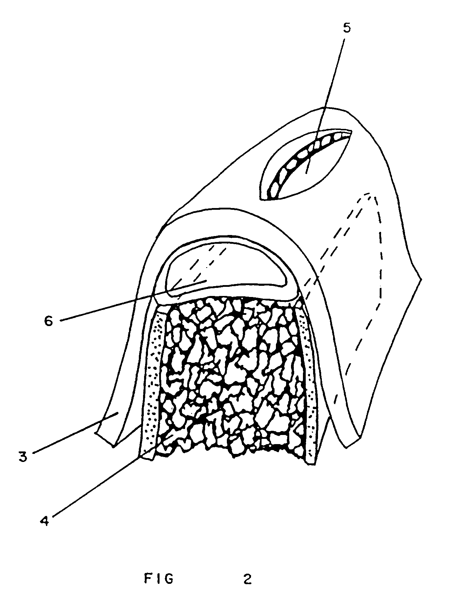

La figure 2 est une vue, également schématique, illustrant le dispositif d'expansion vide inséré entre l'os et la gencive;
La figure 3 illustre de façon schématique, le pré-remplissage du dispositif d'expansion avec un liquide;

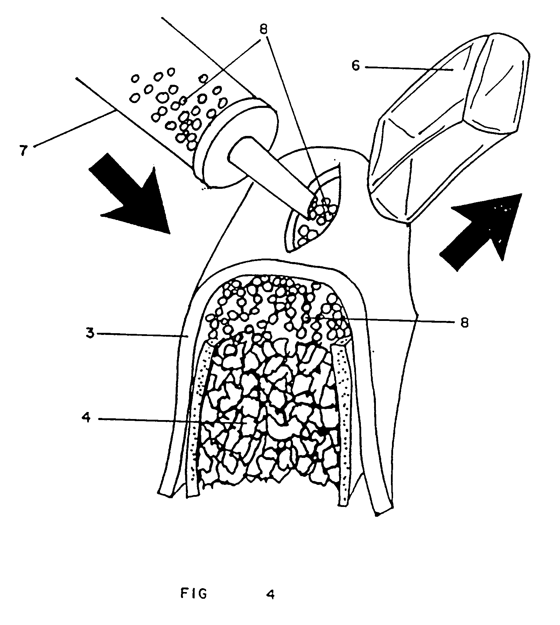

La figure 4 illustre le procédé de retrait du dispositif d'expansion et le placement de l'os lyophilisé dans l'espace vide;
La figure 5 illustre, schématiquement, la fixation d'un implant dans la crête alvéolaire reconstituée;
Les figures 6 et 7 montrent schématiquement des parties essentielles de l'invention;
Les figures 8 et 9 montrent les étapes d'un procédé de réalisation d'une poche préférée selon l'invention.

Comme il a été mentionné plus haut, le dispositif d'expansion 6 de cette invention est fait d'une pellicule de silicone et peut se présenter sous la forme générale rectangulaire, c'est-à-dire d'un parallélépipède, en arc, en demi-arc, en cylindre de petit diamètre, ou encore sous forme de racine dentaire, selon les nécessités spécifiques de chaque cas. A savoir, la forme, les dimensions et les courbes de l'expanseur 6 seront celles qui s'adapteront le mieux au cas spécifique d'un patient.

La fonction du dispositif d'expansion 6 consiste à créer un interstice entre la gencive 3 et l'os érodé 4 afin de ménager un espace pour le nouvel os 4'. Le dispositif d'expansion peut également être utilisé à titre préventif après une extraction, pour éviter une perte osseuse éventuelle.

Le procédé de reconstruction orale consiste à insérer 5 le dispositif d'expansion 6 vide entre la gencive 3 et l'os 4 érodé. Le dispositif d'expansion est ensuite graduellement rempli d'un liquide à l'aide d'une seringue 7, en prenant soin de respecter la capacité d'expansion de la gencive. Après une période de 4 semaines, on retire le dispositif d'expansion 6, laissant ainsi le nouvel espace créé prêt pour recevoir un matériau 8 qui induit la croissance osseuse. Ce matériau 8 peut être de l'os lyophilisé qui induit chimiquement la croissance du nouvel os. A la place de l'os lyophilisé, on peut aussi utiliser de l'hydroxyapatite de calcium, qui induit également la croissance osseuse.

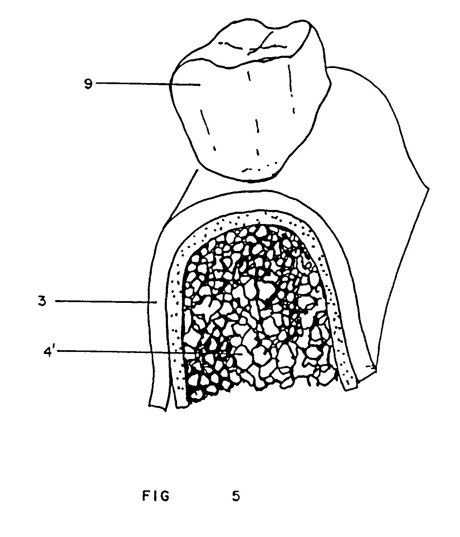

Dans un délai maximum de six mois, la crête alvéolaire est reconstituée et n'importe quel patient peut se soumettre à l'installation d'implants 9 ou prothèses, comme le montre la figure 5.

La présente invention, décrite et illustrée ici, pourra être modifiée de diverses manières dans le cadre de la portée de l'invention définie dans les revendications annexes.

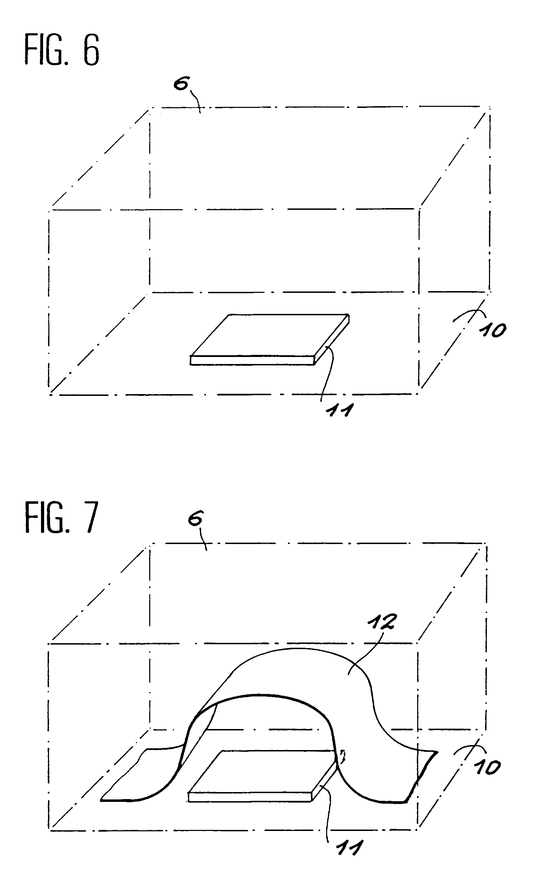

Par rapport au dispositif d'expansion connu le dispositif 6 d'expansion de l'invention comporte, dans la paroi qui le constitue, une surépaisseur. Par exemple, figure 6, sur une portion 10 de pellicule en silicone, ou autre matériau élastique, destinée à former un dispositif 6 d'expansion, on colle, en surépaisseur, une plaque 11, de préférence en un même matériau. Dans un exemple l'épaisseur de la pellicule est de 0,1 mm, celle de la plaque 11 est de 1 mm à 1,5 mm. La surépaisseur peut néanmoins être obtenue par d'autres moyens, par exemple par moulage d'une paroi de la poche à constituer dans un moule contenant une cavité. Puis avec cette portion 10 de pellicule ainsi apprêtée on constitue une poche. Par exemple on assemble divers morceaux de pellicule pour réaliser les formes indiquées: rectangulaires, en arc, en demi arc, en cylindre, ou même en cône pour les racines dentaires. Ce faisant on s'arrange pour que la surépaisseur constituée par la plaque occupe, dans la forme réalisée, une position d'où elle sera accessible dans la bouche du patient. Par exemple cette position est proche de la partie supérieure de la gencive. En pratique, de préférence, dans la poche réalisée, la plaque 11 quand elle est collée n'est plus apparente: elle est fixée à l'intérieur de la poche.

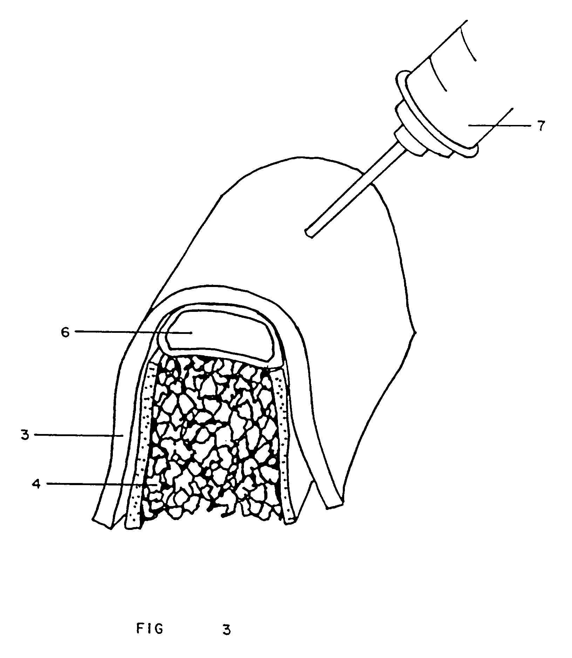

Pour le remplissage graduel du dispositif d'expansion, on approche une seringue, figure 3, de la poche 6. On perce alors la poche 6, à l'endroit de la surépaisseur 11, au travers de la gencive. Et on remplit graduellement la poche avec un liquide. Lorsqu'on retire la seringue, le matériau de la plaque 11 tend à réoccuper, par élasticité, la place de l'orifice créé par la seringue. L'épaisseur de la plaque est telle qu'elle constitue alors une valve naturelle. Elle se referme automatiquement. On n'a plus besoin de canule qui relie la poche à l'extérieur. La poche est seule.

Pour éviter lors du perçage de la poche de transpercer cette poche, on place, figure 7, de préférence, un petit capot 12 de protection directement au dessus de la plaque 11. Le capot peut être en métal, par exemple en acier inoxydable, en matière plastique par exemple en PVC, ou de préférence en nylon de type 6,6. Le capot est concave. Le capot n'est pas une demi coquille complète. Il comporte au moins des ouvertures latérales pour laisser passer le fluide injecté. Il est collé à la pellicule 10 de telle façon à se trouver à la fin à l'intérieur de la poche accroché à la plaque 11. Par suite, quand on perce la poche avec la seringue, l'extrémité de l'aiguille de la seringue vient buter sur le capot à l'intérieur de la poche: il devient impossible de transpercer la poche de part en part. Le capot n'est pas de préférence collé sur tout son pourtour. Le capot est fixé par exemple par deux pattes aboutissant de préférence directement de part et d'autre de la surépaissseur, à la même partie de la paroi de la poche que celle qui porte la surépaisseur. Le capot peut ainsi avoir une forme de demi coque, être concave. Le liquide peut donc s'écouler à l'intérieur de la poche par les côtés non collés du capot.

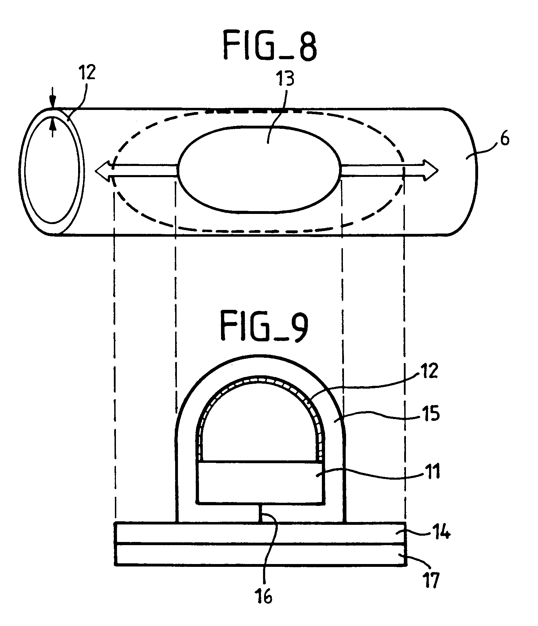

Les figures 8 et 9 montrent un procédé préféré de réalisation d'un dispositif selon l'invention. On commence par fabriquer une poche 6 de forme désirée et dont la figure 8 montre une partie utile, par exemple en forme de tube. La poche 6 est faite d'une couche 12 de silicone soumise à une vulcanisation par cuisson. Dans cette poche 6 on réalise un trou 13 de diamètre égal sensiblement à 4 mm. On réalise ensuite, figure 9, une pastille 14 en silicone vulcanisé d'épaisseur 0,2 mm et de diamètre 8 à 10 mm, une plaque valve 11 en silicone vulcanisé de 1 mm d'épaisseur et de diamètre égal au diamètre du trou 13, ainsi qu'une demie coquille 12 ouverte latéralement en nylon dont le diamètre est aussi égal à celui du trou. On assemble ensuite la demie coquille 12 à la plaque 11 à l'aide d'une bande 15 en silicone cru (non encore vulcanisé). Le collage se produit naturellement et la bande 15 est aboutée sur elle même en dessous de la plaque 14 et 16. La bande 15 a une épaisseur de 0,2 mm. On fait ensuite cuire le tout pour obtenir en un assemblage une valve 14 accrochée à son capot 15 et à sa base 14, mais non encore en place dans la poche 6. Pour effectuer cette mise en place on plaque une couche 17 de silicone cru sous l'assemblage 11-16. De préférence cette couche 17 peut être provisoirement recouverte d'un film plastique qui permet la manutention. On écarte ensuite avec les doigts les bords du trou 13 et, dans l'orifice agrandi, on introduit l'assemblage, capot en avant. En pratique à ce moment le capot vient buter provisoirement sur la paroi de la poche 6 opposée au trou. On enlève alors le film plastique protecteur éventuel. Par pression et massage on vient ensuite coller les bords de la face interne de la poche à l'endroit du trou contre la couche 17. On cuit ensuite le tout pour vulcaniser la couche 17 au contact à la fois de la couche 14 et de la paroi 6. Le montage est alors terminé.

## Revendications

1. Dispositif d'expansion pour reconstruction orale constitué par une poche (6) fermée faite d'une pellicule munie en un endroit (10) d'une surépaisseur (11) et d'une protection contre les perforations de part en part, caractérisé en ce que la protection comprend à l'intérieur de la poche un capot (12) disposé en regard de la surépaisseur, collé directement de part et d'autre au dessus de la surépaisseur mais pas sur tout son pourtour.

2. Dispositif d'expansion, conformément à la revendication 1, caractérisé par le fait que la forme générale de ladite poche (6) est un parallélépipède.

3. Dispositif d'expansion, conformément à la revendication 1, caractérisé par le fait que la forme générale de ladite poche (6) est un arc ou un demi-arc.

4. Dispositif d'expansion, conformément à la revendication 1, caractérisé par le fait que la forme générale de ladite poche (6) est un cylindre.

5. Dispositif d'expansion, conformément à la revendication 1, caractérisé par le fait que la forme générale de ladite poche (6) est celle d'une racine dentaire.

6. Dispositif d'expansion, conformément à l'une des revendications 1 à 5, caractérisé par le fait que la surépaisseur est placée à l'intérieur de la poche.

7. Dispositif selon l'une des revendications 1 à 6, caractérisé par le fait que la protection comporte une demi coquille vulcanisée sur la valve et sur une pastille.

8. Dispositif selon la revendication 7, caractérisé par le fait que la pastille est fixée par vulcanisation à l'intérieur de la poche.

9. Dispositif selon l'une des revendications 1 à 8, caractérisé en ce que le capot est en nylon.

10. Dispositif selon l'une des revendications 1 à 8, caractérisé en ce que le capot est en plastique.

## Claims

1. Expansion device for oral construction, consisting of a sealed pouch (6) made from a film exhibiting an additional thickness (11) at one point (10) and being protected against perforation right through, characterised in that the protection consists of an internal cover portion (12) positioned opposite the area of additional thickness and bonded directly to either side of the area over which it passes, but not bonded over its entire periphery.

2. Expansion device according to claim 1, characterised in that the pouch (6) has the general shape of a parallelepiped.

3. Expansion device according to claim 1, characterised in that the pouch (6) has the general shape of an arc or half-arc.

4. Expansion device according to claim 1, characterised in that the pouch (6) has the general shape of a cylinder.

5. Expansion device according to claim 1, characterised in that the pouch (6) has the general shape of a tooth root.

6. Expansion device according to any of claims 1 to 5, characterised in that the additional thickness is positioned inside the pouch.

7. Device according to any of claims 1 to 6, characterised in that the protection comprises a female mould vulcanised onto the valve and onto a tablet.

8. Device according to claim 7, characterised in that the tablet is installed inside the pouch by vulcanisation.

9. Device according to any of claims 1 to 8, characterised in that the cover portion is made of nylon.

10. Device according to any of claims 1 to 8, characterised in that the cover portion is made of plastic.

## Patentansprüche

1. Expansionsvorrichtung zur oralen Rekonstruktion, die aus einer geschlossenen Tasche (6) besteht, die aus einem Film hergestellt ist, der in einem Bereich (10) eine erhöhte Stelle (11) und einen Schutz gegen durchgehende Perforierungen aufweist, dadurch gekennzeichnet, daß der Schutz im Inneren der Tasche eine Kappe (12) aufweist, die der erhöhten Stelle gegenüber angeordnet ist und direkt beidseits über der erhöhten Stelle, jedoch nicht um deren ganzen Umfang herum, festgeklebt ist.

2. Expansionsvorrichtung nach Anspruch 1, dadurch gekennzeichnet, daß die allgemeine Form der Tasche (6) ein Quader ist.

3. Expansionsvorrichtung nach Anspruch 1, dadurch gekennzeichnet, daß die allgemeine Form der Tasche (6) ein Bogen oder Halbbogen ist.

4. Expansionsvorrichtung nach Anspruch 1, dadurch gekennzeichnet, daß die allgemeine Form der Tasche (6) ein Zylinder ist.

5. Expansionsvorrichtung nach Anspruch 1, dadurch gekennzeichnet, daß die allgemeine Form der Tasche (6) eine Zahnwurzel ist.

6. Expansionsvorrichtung nach einem der Ansprüche 1 bis 5, dadurch gekennzeichnet, daß sich die erhöhte Stelle innerhalb der Tasche befindet.

7. Expansionsvorrichtung nach einem der Ansprüche 1 bis 6, dadurch gekennzeichnet, daß der Schutz eine Halbschale umfaßt, die auf dem Ventil und auf einem Plättchen vulkanisiert ist.

8. Vorrichtung nach Anspruch 7, dadurch gekennzeichnet, daß das Plättchen durch Vulkanisation innerhalb der Tasche befestigt ist.

9. Vorrichtung nach einem der Ansprüche 1 bis 8, dadurch gekennzeichnet, daß die Kappe aus Nylon besteht.

10. Vorrichtung nach einem der Ansprüche 1 bis 8, dadurch gekennzeichnet, daß die die Kappe aus Kunststoff besteht.
